# EUROPEAN PATENT APPLICATION

(11) **EP 4 566 613 A1**
(43) Date of publication of application: **11.06.2025**
(21) Application number: 23850114.2
(22) Date of filing: 02.08.2023
(51) Int. Cl.: A61K 31/7115, A61K 31/42, A61K 31/436, A61K 31/519, A61K 31/57, A61K 38/13, A61K 47/60, A61P 13/10, C12N 15/115

(54) **THERAPEUTIC AGENT FOR HUNNER-TYPE INTERSTITIAL CYSTITIS CONTAINING DNA OLIGONUCLEOTIDE SELECTIVELY BINDING TO IFN-gamma**

(30) Priority: 03.08.2022 JP 2022123809
(71) Applicant: TAGCyx Biotechnologies Inc., Tokyo 1530041 (JP)
(72) Inventor: HARADA, Kaori, Tokyo 153-0041 (JP); MUTO, Susumu, Tokyo 153-0041 (JP)
(74) Representative: RGTH
(86) International application number: PCT/JP2023/028263
(87) International publication number: WO 2024/029560

(57) **Abstract**

Provided is a Hunner-type interstitial cystitis therapeutic agent that can selectively inhibit IFN-γ without risk of biological contamination and can be stored at room temperature. Provided is a Hunner-type interstitial cystitis therapeutic agent or a reagent for test and research, comprising as an active ingredient a DNA oligonucleotide having a nucleotide sequence set forth in any of SEQ ID NO:1 to 3 and selectively binding to IFN-γ. A base, X, in the sequence of the DNA oligonucleotide having the nucleotide sequence set forth in SEQ ID NO: 3 is an artificially produced base, and the artificially produced base may be chemically modified with a low-molecular-weight compound, a medium-molecular-weight compound, a high-molecular-weight compound, a biopolymer, or a biocompatible polymer.

## Description

### [Technical Field]

The present invention relates to a Hunner-type interstitial cystitis therapeutic agent containing a DNA oligonucleotide that selectively binds to interferon-y (IFN-γ).

### [Background Art]

Hunner-type interstitial cystitis is a disease designated as an intractable disease (designated intractable disease 226) by the Minister of Health, Labour and Welfare on May 2014. Although the exact figure of patients with Hunner-type interstitial cystitis in Japan is unclear, according to Report on Public Health Administration and Services by the Ministry of Health, Labour and Welfare, the number of specified medical expenses (designated intractable disease) recipient certificate holders who are patients with Hunner-type interstitial cystitis is increasing from the fiscal year 2015, and was 863 in the fiscal year 2020 (NPL 1). It is conceivable that many of the patients do not have the recipient certificate. The investigation by Society of Interstitial Cystitis of Japan has concluded that around 4500 patients suffer from interstitial cystitis including Hunner-type interstitial and non-Hunner-type in the entire Japan and reported that 45% of the patients (around 2000 patients) suffer from Hunner-type interstitial cystitis. It is known that the ratio of male and female patients with Hunner-type interstitial cystitis is 1:5.6 and the disease is more common in women, especially in middle-aged or elderly people (NPL 2).

Interstitial cystitis is a disease in which idiopathic inflammation occurs in the bladder, resulting in strenuous symptoms such as frequent urination and pain or discomfort in the bladder or the urethra. Interstitial cystitis is classified into the Hunner type and the non-Hunner type according to the disease type. In Hunner-type, peculiar abnormal inflammatory pathological images referred to as Hunner's lesions are observed in the bladder by cystoscopy. Meanwhile, inflammatory lesions are not observed in the bladder in non-Hunner-type interstitial cystitis, which has proved to be a disease that is quite different from Hunner-type. Presently, interstitial cystitis refers to Hunner-type interstitial cystitis.

Any radical therapy for Hunner-type interstitial cystitis has not been discovered yet. Clinical guidelines (NPL 3) exemplify conservative therapy (palliative therapy), oral therapeutic agents, intravesical injection therapy, and surgical treatment including cystoscopic surgery as methods for improving symptoms. Insurance has covered only bladder hydrodistention as therapy for interstitial cystitis until now. Unfortunately, bladder hydrodistention is highly invasive, involves the risk of bladder rupture as an adverse effect, and imposes heavy physical and temporal burdens associated with the practice thereof on patients. Zymso(R) Intravesical Solution 50% (which is injected six times at intervals of two weeks) was newly approved as the only therapeutic agent for interstitial cystitis in Japan in January 2021. Unfortunately, Zymso(R) Intravesical Solution 50% is still disadvantageous in that no one has fully clarified the mechanism in which dimethyl sulfoxide (DMSO), which is the active ingredient thereof, acts on interstitial cystitis, and the therapeutic agent is effective against pain and inflammation to a certain degree, but is short-acting, leading to the repetitive recurrences.

Pathological explanation or dietary intervention is used as palliative therapy. For example, analgesic agents, antidepressants, antiallergic agents, and steroids are used as oral therapeutic agents. The above-mentioned bladder hydrodistention has been widely used as cystoscopic surgery. If Hunner's lesions are observed in the bladder during the practice thereof, electric or laser surgery (ablation) is performed, but surgery repeated for a long term disadvantageously atrophies the bladder. Although the symptoms are observed to be ameliorated by bladder hydrodistention or the ablation of Hunner's lesions in around half of the cases, it is only some cases that are ameliorated for a long term. Many cases therefore require retreatment or additional treatment.

For example, heparin or steroids are used for intravesical injection therapy besides 50% DMSO. Botulinum toxin may be injected into the bladder wall. The total resection of the bladder and urinary diversion are applied to cases in which intolerable symptoms last that are resistant to all the treatments.

### [Citation List]

### [Non Patent Literature]

[NPL 1] Report on Public Health Administration and Services by the Ministry of Health, Labour and Welfare, the Number of Specific Medical Expense (Designated Intractable Disease) Recipient Certificate Holders, Data as of the fiscal years 2015 to 2020) <https://www.nanbyou.or.jp/entry/5354>
[NPL 2] The Japan Intractable Diseases Information Center website <https://www.nanbyou.or.jp/entry/4429>
[NPL 3] Clinical Guideline for Interstitial Cystitis/Bladder Pain Syndrome (Editor: The Society of Interstitial Cystitis of Japan/The Japanese Urological Association), published on April 25, 2019, revised in May 2021.

### [Summary of Invention]

### [Technical Problem]

As mentioned above, a therapeutic agent and a therapy have not been found yet that are effective against Hunner-type interstitial cystitis. Improvement in the QOL (quality of life) of patients requires the development of a highly effective therapeutic agent or a highly effective therapy.

Hunner-type interstitial cystitis is pathologically characterized in the infiltration of lymphocytes, especially B cells and plasma cells, in lesions, and exhibits inflammatory condition due to typical autoimmune disease. The pathogenesis thereof is however still unknown.

It has been revealed that Hunner-type interstitial cystitis is often associated with systemic autoimmune diseases such as Sjogren's syndrome, autoimmune thyroiditis, and systemic lupus erythematosus. It has been reported that an autoantibody to urothelium is detected from blood of patients (Y. Akiyama, et al., International Journal of Urology, 2020, 27, 491-503.). By the gene expression analysis of bladder samples collected from Hunner-type interstitial cystitis patients, the significant increase in gene expression of inflammation-related molecules such as IFN-γ, CXCR3, CXCL9, CXCL10, CXCL11, TNF-α, and TNFSF14 was reported (T. Ogawa, et al., the Journal of Urology, 2010, 183, 1206-1212.). Most of these molecules are IFN-γ and cascade molecules thereof. If considering the fact that the diseases associated with Hunner-type interstitial cystitis are systemic autoimmune diseases, it is conceivable that IFN-γ plays a key role in the pathogenesis of Hunner-type interstitial cystitis, like other autoimmune diseases. However, until now, any therapeutic agent for Hunner-type interstitial cystitis targeting IFN-γ has not been developed.

Previously, as agents that inhibit the action of IFN-γ, antibodies and Janus kinase inhibitors have been developed. However, for example, anti-IFN-γ antibodies have some problems, including that (1) since they are biologics, there is a risk of biological contamination and the like, (2) antigenicity becomes a problem in long-term administration, (3) since they are protein preparations, a cold chain is required for storage and transportation, and (4) since the anti-IFN-γ antibodies have high molecular weights, the anti-IFN-γ antibodies are unsuitable for local administration such as intravesical injection.

With regard to the problem (2) mentioned above, the rate of anti-drug antibody generation for common antibody drugs is said to be about 30%. Therefore, when a long-term treatment is required, a case is often observed in which generation of the anti-drug antibody causes the anaphylactic reaction and making it difficult to continue the treatment.

Furthermore, with regard to the problem (1) mentioned above, since serum and other substances are often used in the manufacturing process of biologics, there is concern about the risk of biological contamination by viruses and the like. With regard to the problem (3), the protein preparation always requires handling at low temperature, which increases the cost of transportation and storage, and also reduces convenience for patients who use the protein preparation.

Four Janus kinase inhibitors including Tofacitinib (product name: Xeljanz^{®}), Baricitinib (product name: Olmient^{®}), Peficitinib (product name: Smyraf^{®}) and Upadacitinib (product name: RINVOQ^{®}) have been applied to and marketed for rheumatoid arthritis, an autoimmune disease. Since these Janus kinase inhibitors are low-molecular-weight compounds that can be manufactured by chemical synthesis, it is thought that there is not the problem caused by being antibodies described above. On the other hand, there are multiple subtypes of Janus kinase, and they are activated by binding to the intracellular domains of multiple cytokine receptors including interleukin 2 (IL-2) receptor, interleukin 4 (IL-4) receptor, interleukin 7 (IL-7) receptor, and interferon-α (IFN-α) receptor, as well as the IFN-γ receptor, and transmit receptor signals.

Therefore, Janus kinase inhibitors may inhibit signaling of not only IFN-γ but also IL-2, IL-4, IL-7, IFN-α, and the like (Yvan Jamilloux, et al., Autoimmunity Reviews, 2019, 18, 11, 102390). This is cause for concern about safety in long-term administration, and it suggests that unexpected adverse effects due to susceptibility to infection may be exhibited.

The present invention has been completed in view of such situation, and an object of the present invention is to provide a Hunner-type interstitial cystitis therapeutic agent that can selectively inhibit IFN-γ as a target substance without risk of biological contamination and can be stored at room temperature.

### [Solution to Problem]

In order to solve the problems mentioned above, a Hunner-type interstitial cystitis therapeutic agent of the present invention containing a DNA oligonucleotide as an active ingredient adopts the following aspects.

The first aspect of the present invention provides a Hunner-type interstitial cystitis therapeutic agent containing a DNA oligonucleotide having a nucleotide sequence set forth in any of SEQ ID NO:1 to 3 and selectively binding to interferon-y (IFN-γ). The DNA oligonucleotide of this aspect exerts a therapeutic effect on Hunner-type interstitial cystitis by selectively binding to IFN-γ and inhibiting the activity thereof.

The nucleotide sequence set forth in SEQ ID NO:2 is a sequence in which an oligonucleotide comprising natural bases of 9 residues is added to a 3' end of the nucleotide sequence set forth in SEQ ID NO:1.

The nucleotide sequence set forth in SEQ ID NO:3 is a sequence in which a 53rd base from the 5' end of the nucleotide sequence set forth in SEQ ID NO:2 was replaced with any base.

In the first aspect described above of the present invention, a base X in a sequence of the DNA oligonucleotide having the nucleotide sequence set forth in SEQ ID NO:3 may be an artificially produced base, and the artificially produced base may be chemically modified with a low-molecular-weight compound.

The low-molecular-weight compound in the first aspect described above has a molecular weight of about 200 to 1000. Examples of a candidate therefor include an anti-inflammatory compound selected from glucocorticoid, tacrolimus, sirolimus, cyclosporine, methotrexate and leflunomide.

In the first aspect described above of the present invention, a base X in a sequence of the DNA oligonucleotide having the nucleotide sequence set forth in SEQ ID NO:3 may be an artificially produced base, and the artificially produced base may be chemically modified with a medium-molecular-weight compound, a high-molecular-weight compound, a biopolymer, or a biocompatible polymer. The medium-molecular-weight compound in this aspect has a molecular weight of about 1000 to 20000, and the high-molecular-weight compound in this aspect has a molecular weight of about 20000 to 400000.

The high-molecular-weight compound in the aspect described above may be any biocompatible large molecule with a molecular weight of 20000 or more. Examples of medium-molecular-weight compounds or high-molecular-weight compounds in the present aspect include, but are not limited to, PEGs, bipolar polymers, oligosaccharides, fat-soluble polymers, peptides, oligonucleotides, and antibodies. Antibodies belong to high-molecular-weight compounds, while PEGs, bipolar polymers, oligosaccharides, fat-soluble polymers, peptides, and oligonucleotides belong to medium-molecular-weight compounds or high-molecular-weight compounds, depending on their molecular weight. The molecular weight of a medium-molecular-weight compound or a high-molecular-weight compound is expressed as an average molecular weight defined by the number average molecular weight (Mn) or the weight average molecular weight (Mw).

The second aspect of the present invention is a therapeutic agent containing a DNA oligonucleotide as an active ingredient having a nucleotide sequence set forth in any of SEQ ID NO:1 to 3 and selectively binding to dog IFN-γ and cat IFN-γ, wherein the therapeutic agent is used for a disease selected from diseases related to dog IFN-γ and cat IFN-γ, lower urinary tract diseases due to the bladders of dogs and cats, and autoimmune diseases of dog and cat. Examples of lower urinary tract diseases due to the bladder of dogs and cats include idiopathic cystitis.

In the second aspect described above, the base X in the sequence of the DNA oligonucleotide having the nucleotide sequence set forth in SEQ ID NO: 3 may be an artificially produced base. The artificially produced base may be chemically modified with a low-molecular-weight compound. The low-molecular-weight compound in this aspect has a molecular weight of about 200 to 1000. Examples of a candidate therefor include an anti-inflammatory compound selected from glucocorticoid, tacrolimus, sirolimus, cyclosporine, methotrexate and leflunomide.

In the second aspect described above, the base X in the sequence of the DNA oligonucleotide having the nucleotide sequence set forth in SEQ ID NO: 3 may be an artificially produced base. The artificially produced base may be chemically modified with a medium-molecular-weight compound, a high-molecular-weight compound, a biopolymer, or a biocompatible polymer. The medium-molecular-weight compound in this aspect has a molecular weight of about 1000 to 20000, and the high-molecular-weight compound in this aspect has a molecular weight of about 20000 to 400000. The high-molecular-weight compound in the aspect described above may be any biocompatible large molecule having a molecular weight of 20000 or more. Examples of the medium-molecular-weight compound or the high-molecular-weight compound in this aspect include, but not limited to, PEG, bipolar polymers, oligosaccharides, fat-soluble polymers, peptides, oligonucleotides, and antibodies. Antibodies belong to the high-molecular-weight compound. PEG, bipolar polymers, oligosaccharides, fat-soluble polymers, peptides, and oligonucleotides belong to the medium-molecular-weight compound or the high-molecular-weight compound depending on the molecular weight thereof.

The third aspect of the present invention is a reagent for test and research, comprising a DNA oligonucleotide as an active ingredient having a nucleotide sequence set forth in any of SEQ ID NO: 1 to 3 and selectively binding to IFN-γ.

In the third aspect described above, the base X in the sequence of a DNA oligonucleotide having a nucleotide sequence set forth in SEQ ID NO: 3 may be an artificially produced base. The artificially produced base may be chemically modified with a low-molecular-weight compound. The low-molecular-weight compound in this aspect has a molecular weight of about 200 to 1000. Examples of a candidate therefor include an anti-inflammatory compound selected from glucocorticoid, tacrolimus, sirolimus, cyclosporine, methotrexate and leflunomide.

In the third aspect described above, the base X in the sequence of a DNA oligonucleotide having a nucleotide sequence set forth in SEQ ID NO: 3 may be an artificially produced base. The artificially produced base may be chemically modified with a medium-molecular-weight compound, a high-molecular-weight compound, a biopolymer, or a biocompatible polymer. The medium-molecular-weight compound in this aspect has a molecular weight of about 1000 to 20000, and the high-molecular-weight compound in this aspect has a molecular weight of about 20000 to 400000. The high-molecular-weight compound in the aspect described above may be any large molecule having a molecular weight of 20000 or more. Examples of the medium-molecular-weight compound or the high-molecular-weight compound in this aspect include, but not limited to, PEG, bipolar polymers, oligosaccharides, fat-soluble polymers, peptides, oligonucleotides, and antibodies. Antibodies belong to the high-molecular-weight compound. PEG, bipolar polymers, oligosaccharides, fat-soluble polymers, peptides, and oligonucleotides belong to the medium-molecular-weight compound or the high-molecular-weight compound depending on the molecular weight thereof.

### [Advantageous Effects of Invention]

The DNA oligonucleotide having the nucleotide sequence according to the present invention selectively binds to IFN-γ. The DNA oligonucleotide can selectively inhibit the activity of IFN-γ thereby. Also, according to a Hunner-type interstitial cystitis therapeutic agent containing a DNA oligonucleotide having a nucleotide sequence of the present invention, its production is made without a risk of biological contamination by viruses and the like, since there is no need to use serum or other biological substances in its production. Further, the DNA oligonucleotide having a nucleotide sequence of the present invention can be stored at room temperature, which is advantageous compared to conventional methods in terms of transportation and storage costs and can improve convenience for patients who use it. The Hunner-type interstitial cystitis therapeutic agent containing the DNA oligonucleotide having the nucleotide sequence of the present invention can be administered by intravesical injection due to the molecular weight thereof.

While the DNA oligonucleotide of the present invention does not inhibit the signaling of IL-2, IL-4, IL-7, IFN-α, and the like, the DNA oligonucleotide inhibits only the action of IFN-γ. According to the Hunner-type interstitial cystitis therapeutic agent containing as an active ingredient the DNA oligonucleotide having a nucleotide sequence of the present invention, unanticipated adverse effects due to susceptibility to infection can thus be reduced compared to Janus kinase inhibitors and other therapeutic agents even when administered for a long term. Further, when compared to anti-IFN-γ antibodies, the DNA oligonucleotide of the present invention is less antigenic than antibodies, which makes it an agent that can be used for a long term. Furthermore, the DNA oligonucleotide of the present invention is manufactured without a risk of biological contamination, and can be stored at room temperature.

### [Brief Description of Drawings]

[Fig. 1] Fig. 1 shows a change in the number of hairs on a grafted skin patch before and after administration of the DNA oligonucleotide of an embodiment of the present invention.
[Fig. 2A] Fig. 2A shows the experimental results of MHC class I expression in the dermal sheath cup by administration of the DNA oligonucleotide according to an embodiment of the present invention.
[Fig. 2B] Fig. 2B shows the experimental results of MHC class I expression in the outer root sheath by administration of the DNA oligonucleotide according to an embodiment of the present invention.
[Fig. 3A] Fig. 3A shows the experimental results of MHC class II expression in the connective tissue sheath by administration of the DNA oligonucleotide according to an embodiment of the present invention.
[Fig. 3B] Fig. 3B shows the experimental results of MHC class II expression in the outer root sheath by administration of the DNA oligonucleotide according to an embodiment of the present invention.
[Fig. 4A] Fig. 4A shows the results of the effect on mouse IFN-γ-induced STAT1 phosphorylation by the addition of surrogate aptamer to L929 mouse fibroblasts in an embodiment of the present invention.
[Fig. 4B] Fig. 4B shows the results of the effect on mouse IFN-γ-induced STAT1 phosphorylation by the addition of negative control DNA to L929 mouse fibroblasts.
[Fig. 5A] Fig. 5A shows a suppression of an increase in the urinary frequency in an experiment on the effect of a surrogate aptamer on a mouse interstitial cystitis model according to an embodiment of the present invention.
[Fig. 5B] Fig. 5B shows a suppression of a rise in sensitivity to pelvic nociception in an experiment on the effect of a surrogate aptamer on a mouse interstitial cystitis model according to an embodiment of the present invention.
[Fig. 6] Fig. 6 shows the results of histopathological evaluation of the bladders regarding the effect of a surrogate aptamer on a mouse interstitial cystitis model according to an embodiment of the present invention.
[Fig. 7A] Fig. 7A shows the comparison of the expression levels of mRNAs in the bladder tissues in an experiment on the effect of a surrogate aptamer on a mouse interstitial cystitis model according to an embodiment of the present invention. The expression levels of inflammatory cytokines (IFN-γ and TNF-α) and pain-causing substances (pre-SP and NGF) in the bladder tissue in the surrogate aptamer-treated group were reduced to normal levels, which are equivalent to those of the Normal group.
[Fig. 7B] Fig. 7B shows graphs quantifying the expression levels of the mRNAs shown in Fig. 7A.
[Fig. 8] Fig. 8 shows the binding of a DNA oligonucleotide according to an embodiment of the present invention to dog IFN-γ and cat IFN-γ.

### [Description of Embodiments]

Hereinafter, embodiments of a Hunner-type interstitial cystitis therapeutic agent containing an DNA oligonucleotide as an active ingredient of the present invention will be described.

With regard to a drug that targets IFN-γ, Emapalumab, an anti- IFN-γ antibody, has been approved by FDA in 2018 for hemophagocytic lymphohistiocytosis, which is an intractable autoimmune disease until now, and it is marketed under the trade name Gamifant.

However, as described above, anti-IFN-γ antibodies have problems such as biological contamination and other risks due to being biologics, antigenicity in long-term administration, unsuitability for topical administration including transdermal administration or transmucosal administration due to the high molecular weight thereof, and conditions for storage and transportation due to being protein preparations. Therefore, the creation of a therapeutic agent has been desired that solves these problems and inhibits IFN-γ effectively.

Therefore, the present inventors attempted to develop an IFN-γ inhibitor using the DNA oligonucleotide of the present invention as a DNA aptamer as a means to solve the problems described above. The DNA aptamer is a ligand molecule which forms a secondary structure or a three-dimensional structure of a single-stranded DNA oligonucleotide by forming a complementary strand between complementary sequences within the DNA oligonucleotide molecule, and binds specifically and strongly to a target molecule by its steric structure. While the binding of the DNA aptamer having a specific sequence can inhibit or suppress the activity of target molecules, the binding also enables the enhancement thereof. Although DNA aptamers are smaller than antibodies, with a molecular weight of about one-tenth or less than that of antibodies, they have high affinity and high target selectivity comparable to those of antibodies. Therefore, an IFN-γ inhibitor using a DNA aptamer can minimize the occurrence of adverse effects due to off-target. It is thought that DNA aptamers are a suitable modality as a means to solve the problem, since DNA aptamers can be produced through chemical synthesis. As used herein, "selectively binds to IFN-γ" includes strong and specific binding of the DNA oligonucleotide of the present embodiment as a DNA aptamer to IFN-γ, which is a target substance.

Since DNA aptamers form more compact three-dimensional structures than antibodies, DNA aptamers enable topical administration such as transdermal administration or transmucosal administration.

DNA aptamers have the following advantages and are expected to be useful: (1) with a relatively small molecular weight, administration by transdermal formulations such as ointments and patches or transmucosal formulations may be possible; (2) as chemically synthesized products, a risk of biological contamination is low; (3) generally antigenicity is low; (4) due to being DNA, sufficient stability is secured at room temperature under nuclease-free and almost neutral conditions; and (5) with almost no inhibitory activity to cytochrome P450, a drug-metabolizing enzyme, there is no effect on concomitant agents, and the like. Further, DNA aptamers do not have a problem of generation of anti-drug antibody, which is one of the problems that occurs when long-term treatment with the antibody is necessary, and therefore, long-term administration is possible.

As a specific method to treat diseases using the DNA aptamer, the method is assumed to treat autoimmune diseases or diseases considered to result mainly from the overproduction of IFN-γ by neutralizing IFN-γ through administration of the aptamer itself or a modified form of the aptamer.

The present inventors have found a DNA aptamer having a sequence of SEQ ID NO: 1 in Table 1 including two artificial bases, in the nucleotide sequence, that are Ds (7-(2-thienyl)imidazo[4,5-b]pyridine) as a DNA aptamer that can bind to human IFN-γ with high affinity to inhibit the activity thereof.

Even though the DNA aptamers exhibit strong activity *in vitro,* the DNA aptamers do not necessarily express activity *in vivo* for the following reason: DNA aptamers are rapidly degraded by nucleases in biological tissue. Accordingly, the present inventors have found that a DNA aptamer having a nucleotide sequence of SEQ ID NO: 2 in Table 1 wherein a natural nucleotide sequence of nine residues is added to the 3' end of the nucleotide sequence of SEQ ID NO: 1 has nuclease-resistance and is stable in biological tissue. It was confirmed that the DNA aptamer having the nucleotide sequence of SEQ ID NO: 2, including two Ds, exhibits efficacy in an autoimmune humanized mouse alopecia areata model, into which human scalp tissue is transplanted, is stable in biological tissue, and can inhibit IFN-γ (Japanese Patent Application No. 2021-166794). In the sequence set forth in SEQ ID NO: 2, the 53rd base from the 5' end is substituted with any base, X, to obtain a sequence of SEQ ID NO: 3. Polyethylene glycol (PEG) is added to the base X to obtain a PEG-modified form of a DNA aptamer having the sequence of SEQ ID NO: 3. It was confirmed by SPR (surface plasmon resonance) that the PEG-modified form is capable of binding to IFN-γ. This has shown that the modification of the base, X, does not affect the IFN-γ-binding activity of the DNA aptamer having a sequence of SEQ ID NO: 3, namely that the DNA aptamer having the sequence of SEQ ID NO: 3 has IFN-γ inhibitory activity equivalent to that of the DNA aptamer having the sequence of SEQ ID NO: 2.

In the present embodiment, the DNA oligonucleotides having the nucleotide sequences listed in Table 1 are used as the DNA aptamers. The present embodiment comprises the use of the DNA oligonucleotides having the nucleotide sequences listed in Table 1 as the Hunner-type interstitial cystitis therapeutic agents.

**[Table 1]**

| SEQ ID NO: | Sequence (5' → 3') |
|---|---|
| 1 | CCCGCCCGGGTCCGCGAAGCGGTAGGT**Ds**TGGGCTAGGC**Ds**GCTGGCGGG |
| 2 | CCCGCCCGGGTCCGCGAAGCGGTAGGT**Ds**TGGGCTAGGC**Ds**GCTGGCGGGCGCGAAGCG |
| 3 | CCCGCCCGGGTCCGCGAAGCGGTAGG**Ds**TGGGCTAGGC**Ds**GCTGGCGGGCGCXAGCG |
| 4 | GGCCGGTACCCA**Ds**CCACAGTTTAT**Ds**GTTGTACTAGTTTTGCAGGGTCTGGCCCGCGAAGCG |

The nucleotide sequence set forth in SEQ ID NO:2 of Table 1 is a sequence in which an oligonucleotide comprising natural bases of 9 residues (5'-CGCGAAGCG-3') (mini-hairpin sequence) is added to the 3' end of the sequence set forth in SEQ ID NO:1. The nucleotide sequence set forth in SEQ ID NO:3 of Table 1 is a sequence in which the 53rd base from the 5' end of the sequence set forth in SEQ ID NO:2 was replaced with any base X. The X represents any natural base, any non-natural base or a modified base, or a modified base to which a low-molecular-weight compound, a peptide, an oligonucleic acid, an oligosaccharide, a protein or the like, or a high-molecular-weight compound used in vivo (biopolymer) or a biocompatible polymer is bound. Details of the nucleotide sequence of SEQ ID NO: 4 will be described below.

Examples of the high-molecular-weight compound to be bound to the modified base include polyethylene glycol (PEG) having a molecular weight of 20000 or more and any biocompatible large molecule having a molecular weight of 20000 or more. A biocompatible polymer is a chemically synthesized compound that is not normally used in vivo and safe enough to be placed in vivo without causing inflammation or toxic reactions. Examples of medium-molecular-weight compounds include peptides, oligonucleic acids, oligosaccharides, proteins, PEGs, and any biocompatible polymers with a molecular weight of 1000 or more and less than 20000.

As functional groups for modification, azido group (-N₃), amino group (-NH₂), carboxyl group (-COOH) or its active ester, alkynyl group (-CC) or a cyclic structure having an alkynyl structure, formyl group (-CHO), hydrazide group (-NH-NH₂), hydroxyl group (-OH), thiol group (-SH), cyano group (-CN), vinyl group (-CHCH₂) and maleimide group can be used.

As used herein, "natural base" refers to either adenine, guanine, cytosine, or thymine. As used herein, "non-natural base" refers to a base that is artificially synthesized having properties similar to the natural base, and is sometimes referred to as "artificial base" herein. As used herein, "modified base" refers to a base to which a side chain structure with one or more functional groups activated for modification is added, and is a kind of "artificially produced base". Examples of modifications include methylation, deamination, atomic place exchange, thiolation of oxygen in phosphate site, and introduction of a water-soluble or fat-soluble substituent group into the base portion of a natural base. Specifically, the examples include modified pyrimidines, modified purines, and other heterocyclic bases. Ds in SEQ ID NO:1 to 3 represents 7-(2-thienyl)imidazo[4,5-b]pyridine, an artificial base. As an artificial base, in addition to Ds itself, a base with a side chain introduced into Ds may be used. Hereafter, in the present embodiment, a DNA aptamer having a sequence set forth in SEQ ID NO:1, 2, 3, or 4 of Table 1 will be referred to as "Aptamer 1", "Aptamer 2", "Aptamer 3" or "Aptamer 4" respectively.

With respect to the usefulness of the DNA aptamer of the present embodiment, it was observed herein that when a DNA aptamer listed in Table 1 (aptamer 2) was injected intradermally into the transplanted skin of an immune-tolerance mouse of autoimmune hair loss model which was transplanted with human scalp skin patches, regeneration of hair was promoted, and suppression of further hair loss was confirmed (Example 4). Details will be described later.

Pathological analysis of the mechanism of activity expression of Aptamer 2 in this model revealed that Aptamer 2 almost completely inhibited expression of MHC class I and II. Detailed results will be described later. That is, it is thought that by inhibiting activity of IFN-γ, Aptamer 2 inhibited expression of MHC class I and II, the source of expression of autoimmunity, and improve autoimmunity, thus improving the symptoms of alopecia areata. This indicates that Aptamer 2 is effective not only against alopecia areata but also against Hunner-type interstitial cystitis, considered to result mainly from overproduction of IFN-γ, and that providing an IFN-γ inhibitor containing the DNA aptamer could be a means to solve the problems.

It needs to be confirmed in a disease animal model before the certification of the efficacy of DNA aptamers in humans whether the DNA aptamers that inhibit the activity of IFN-γ are effective in the treatment of Hunner-type interstitial cystitis. Examples of the known animal model that has been established until now, and is the most similar in clinical condition of Hunner-type interstitial cystitis to humans include a mouse autoimmune interstitial cystitis model (model using a transgenic mouse in which the OVA antigen is expressed in the bladder (URO-OVA mouse)) (Y. Akiyama, et al., Am. J. Physiol. Renal Physiol., 2021, 320, F174-182: hereinafter referred to as "Reference Literature 1".). Unfortunately, the above-mentioned DNA aptamers that specifically bound to human IFN-γ did not exhibit binding activity to mouse IFN-γ. The efficacy of the above-mentioned DNA aptamers cannot, therefore, be confirmed in the mouse model.

Accordingly, the present inventors have examined the acquisition of an aptamer that is highly similar in physical properties to the above-mentioned DNA aptamers, and binds to mouse IFN-γ to inhibit the activity thereof as a surrogate aptamer.

The surrogate aptamer needs to satisfy the following criteria to be highly similar in physical properties to the above-mentioned DNA aptamers.
(1) The aptamer is a DNA aptamer.
(2) The aptamer contains two artificial bases, Ds, in the nucleotide sequence thereof, and has natural bases as the other bases.
(3) The number of bases of the aptamer is within the range of 57 residues ± 10% (51 to 62 residues).
(4) The aptamer has a mini-hairpin sequence of nine residues on the 3' end of the nucleotide sequence thereof.

Since the aptamer satisfying the above-mentioned criteria is similar in structure to the above-mentioned DNA aptamers, the aptamer is assumed to be also similar in physical properties.

A search for the surrogate aptamer satisfying the above-mentioned criteria enables obtaining a DNA aptamer having a sequence listed in SEQ ID NO: 4 of Table 1, having residues with 62 bases, containing two Ds, and having a mini-hairpin sequence on the 3' end thereof (aptamer 4) (Example 5). Details thereof will be described below. The obtained DNA aptamer had a KD value of 2.47 nM, which indicates the capability to bind to mouse IFN-γ, and is around one hundredth as high as the capability of the aptamer 2 to bind to human IFN-γ (KD value: 33 pM). The aptamer having five times the molarity of mouse IFN-γ was added to the mouse IFN-γ to inhibit mouse IFN-γ competitively, resulting in suppressing the activity of mouse IFN-γ almost completely. This result confirmed that the DNA aptamer obtained by the search has sufficient activity to function as a surrogate aptamer.

The obtained surrogate aptamer was intravesically administered to the autoimmune interstitial cystitis model (URO-OVA model) mice described in Reference Literature 1. The surrogate aptamer consequently suppressed an increase in urinary frequency and a rise in sensitivity to pelvic nociception significantly in the mice, compared to the control PBS-treated group, and exhibit a high effect of suppressing the development of cystitis(Example 6).

The results of histopathological evaluation of the bladders has shown that since the inflammation score of the bladder in the aptamer-treated group is significantly low compared to that of the PBS-treated group, and is not different from that of Normal group, the administration of the surrogate aptamer suppressed bladder inflammation. The results of mRNA expression analysis in bladder tissue have shown that the administration of the surrogate aptamer suppressed the expression of inflammatory cytokines such as IFN-γ and TNF-α induced by IFN-γ stimulation, and additionally the expression of pre-SP and NGF, which are pain-causing substances. It has been strongly suggested that the surrogate aptamer exhibited the effect of suppressing bladder inflammation due to the action of inhibiting the activity of IFN-γ in the tissue (Example 6).

Since the DNA aptamer of the present embodiment exhibits the inhibitory action on human IFN-γ in human tissue, and is highly similar in physical properties to the surrogate aptamer, it has been inferred that the DNA aptamer has the action of suppressing the development of Hunner-type interstitial cystitis in humans, and is useful as the Hunner-type interstitial cystitis therapeutic agent like the surrogate aptamer.

As a DNA aptamer, DNA oligonucleotides having any of the sequences listed in Table 1 can be used as they are, or it is also possible to use those modified at a site that does not affect activity of the DNA aptamer. Examples of the modified form of the DNA aptamer include a DNA aptamer chemically bound to a medium-molecular-weight or high-molecular-weight compound such as PEG, a peptide, or an oligonucleotide, a multimer into which the same DNA aptamers are formed by a chemical process, and a DNA aptamer having a partially converted or modified sequence. When a DNA oligonucleotide of the present embodiment is modified and used as a DNA aptamer, the base portion is preferred as the modification portion. An artificial base or a modified base can be modified using existing methods, and the 3' and 5' ends can also be modified.

PEG-modified forms of DNA aptamers are utilized for improving Pharmacokinetics (PK)-Pharmacodynamics (PD) Profile that is commonly used for proteins, peptides, oligonucleic acids including aptamers to achieve improved pharmacokinetics, and a number of PEG-modified aptamers have been developed. It is known that if binding activity of a PEG-modified aptamer to a target protein is retained, it shows the same level of activity in the body as the aptamer before PEG modification, and there is almost no toxic expression due to the PEG modification (C. Simone Fishburn, Journal of Pharmaceutical Sciences, 2008, 97, 10, 4167-4183; Katarina D. Kovacevic, et al., Advanced Drug Delivery Reviews, 2018, 134, 36-50).

As drug formulations for systemic administration, formulations can be prepared as an injectable formulation in vials containing lyophilized powders, vials containing aptamer solution, and pre-filled syringes.

The DNA aptamer of the present embodiment can be produced as a preparation for inhalation by placing a nanoparticle adsorbing or containing the DNA aptamer or a solution thereof, or a powder of the DNA aptamer granulated to an appropriate size with a granulating material in an inhalation device.

The DNA aptamer of the present embodiment can be used as an eye drop by dissolving it as it is in a suitable solvent such as a buffer solution utilizing its high water-solubility.

Examples of conceivable methods for topical administration include a method for transmucosal administration. The DNA aptamers of the present embodiment are dissolved in a solvent such as a highly biocompatible buffer solution to be available as an agent to be transmucosally administered such as an agent to be intravesically administered.

As preparations for injection, preparations for inhalation and eye drops, it is possible to use them in a form that the DNA aptamer of the present embodiment is encapsulated or bonded in nanoparticles such as fatty nanoparticles, nanoparticles of biodegradable polymers such as PLGA (Polylactic-co-Glycolic Acid), gold nanoparticles, and then dispersed or dissolved in physiological saline solution, physiological buffer solution, and the like.

The DNA aptamer of the present embodiment can be applied as a topical transdermal agent such as a solution, an ointment, a cream, a lotion, a milky lotion, an emulsion, a gel, a biodegradable microneedle, or a poultice.

In the process of manufacturing a transdermal administration formulation, as an absorption enhancer, lower alcohols such as ethanol, polyhydric alcohols such as ethylene glycol, fatty acids, esters such as ethyl acetate, surfactants, and ionic liquids and the like may be used. For the manufacturing of the transdermal administration formulation, manufacturing process in which biodegradable polymers such as polylactic acid or liposomes are used for making nanoparticles is applicable, and these processes can be combined as appropriate depending on purposes.

The DNA aptamer of the present embodiment can also be used as an administration preparation using a device compatible with physical transdermal absorption enhancement methods such as lontophoresis, Electroporation, Thermalporation, Sonophoresis, a Microneedle array patch, a Needleless syringe, and a micropump.

It was confirmed that the DNA aptamer of the present embodiment also binds to
dog IFN-γ and cat IFN-γ besides human IFN-γ (Example 7). Details thereof will be described below. The overproduction of IFN-γ in dogs and cats is assumed to be involved in idiopathic cystitis, which is one of the lower urinary tract diseases resulting from the bladders of dogs and cats. The results of Example 7 can conclude that the DNA aptamer of the present embodiment has inhibitory activity for the dog IFN-γ and cat IFN-γ activity. It is conceivable from this that the DNA aptamer has the activity of suppressing the development of idiopathic cystitis, which is one of the lower urinary tract diseases resulting from the bladders of dogs and cats. The DNA aptamer of the present embodiment is available as a therapeutic agent for idiopathic cystitis, diseases related to IFN-γ, and autoimmune disease in dogs and cats.

Since the DNA aptamer of the present embodiment can inhibit IFN-γ selectively, the DNA aptamer is available as a research reagent for experiment in which IFN-γ is involved. For example, regardless of *in vitro* or *in vivo,* the possibility whether IFN-γ is involved in a focused physiological phenomenon can be evaluated and examined by the test using the DNA aptamer of the present embodiment, to consider the cause of the physiological phenomenon. The DNA aptamer of the present embodiment is added as a reagent to cell culture medium or administered to animals to be available for a wide variety of tests including reaction systems in which IFN-γ is inhibited.

### Example 1: Synthesis of DNA aptamer

Aptamer 1 and Aptamer 2 were chemically synthesized by the methods described in PCT International Publication No. WO 2013/073602 and PCT International Publication No. WO 2016/143700.

### Example 2: Example of synthesis of Aptamer 3

Aptamer 3 was synthesized by introducing Amino-Modifier C6-dT Amidite to the position of X in the sequence of SEQ ID NO:3 using the methods described in PCT International Publication No. WO 2013/073602 and PCT International Publication No. WO 2016/143700. For other substituents of X, they can be synthesized by using commercially available artificial base or modified base amidites.

### Example 3: Synthesis of PEG-modified DNA aptamer

With the base of X produced in Example 2, Aptamer 3 (1 eq) having a primary amine side chain and commercially available NHS-PEG (40000) (1.5 eq) were mixed in a phosphate buffer at pH 7 to 8 and the mixture was stirred for 1 day at room temperature. The reaction solution was concentrated and the resulting modified form was purified by reversed phase HPLC to obtain a PEG-modified form of Aptamer 3. It was confirmed that the obtained PEG-modified form of Aptamer 3 retained its ability to bind to IFN-γ using a SPR (Surface Plasmon Resonance).

### Example 4: Confirmation of therapeutic effect using humanized mouse model of alopecia areata

### Step 1 Creation of humanized mouse model of alopecia areata

A humanized mouse of the alopecia areata model induced by an intradermal injection of human activated lymphocytes into the scalp skin transplanted to the mice was prepared based on the method described in A. Gilhar, et al., Journal of Investigative Dermatology, 2013, 133, 3, 844-847.

### Step 2

The prepared humanized model mouse of alopecia areata were divided into three groups, and each group was treated with Vehicle (PBS), Dexamethasone + Minoxidil (Positive control), and Aptamer 2. To the Vehicle group, 15 µL of PBS was intradermally administered to the transplanted skin once every 2 days. To the group treated with aptamer 2 (hereafter referred to as the "aptamer-treated group"), a 15 µL of aptamer 2 solution in PBS was intradermally administered to the transplanted skin once every 2 days, and the concentration of the aptamer 2 solution was gradually increased from 12 nM to 300 nM during 143 days. To the group treated with Dexamethasone + Minoxidil, 40 µL of dosing solution containing 2 mg of Dexamethasone and 5% Minoxidil was topical applied to the transplanted skin once daily.

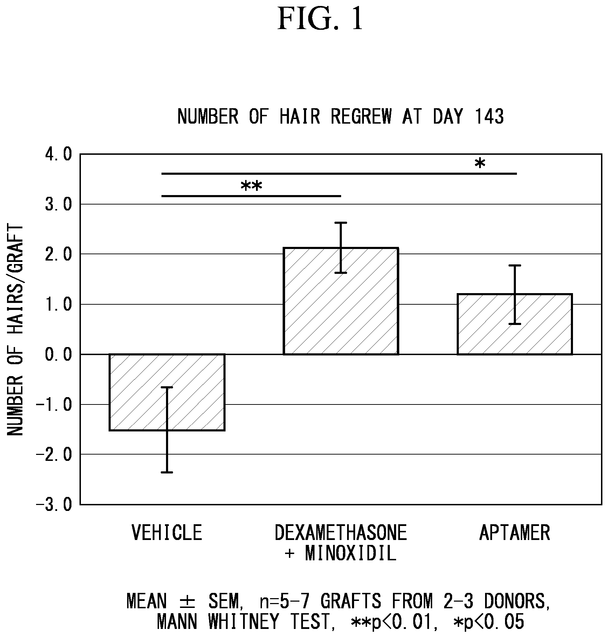

The results after administration in Example 4 are shown in Fig. 1. Fig. 1 shows a change in the number of hairs on the grafted skin patch before and 143 days after the administration, with the vertical axis showing the change in the number of hairs per grafted skin patch. In the Vehicle group ("Vehicle" in Fig. 1), further hair loss was progressed during the PBS administration period, while in the Positive control group ("Dexamethasone + Minoxidil" in Fig. 1) and the Aptamer-treated group ("Aptamer" in Fig. 1), further hair loss was suppressed and also hair regeneration was observed.

The results of pathological analysis of the hair follicle tissue showed a significant inhibition of CD8-positive T-cell infiltration in the Positive control group and the Aptamer-treated group. This suggests that in the Positive control group and the Aptamer-treated group, inhibition of inflammatory reaction suppressed hair loss progression and promoted hair regeneration.

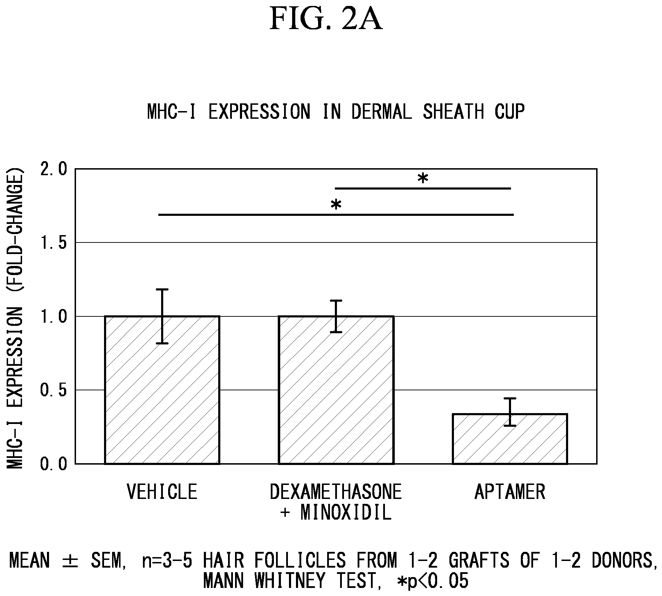

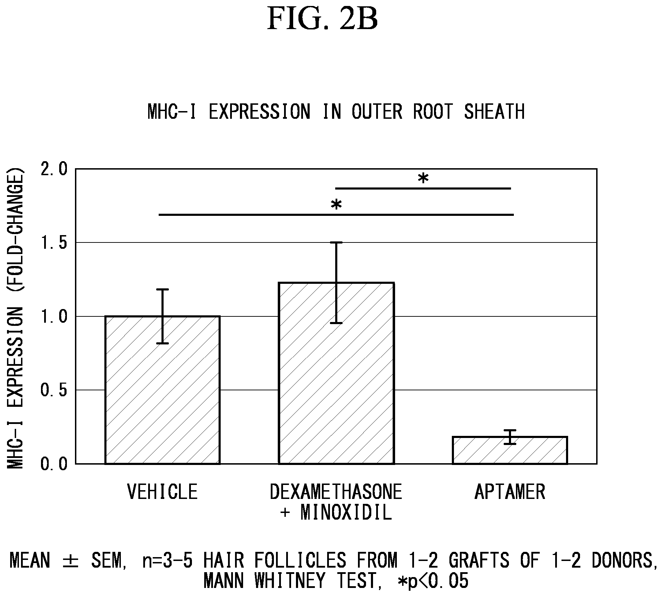

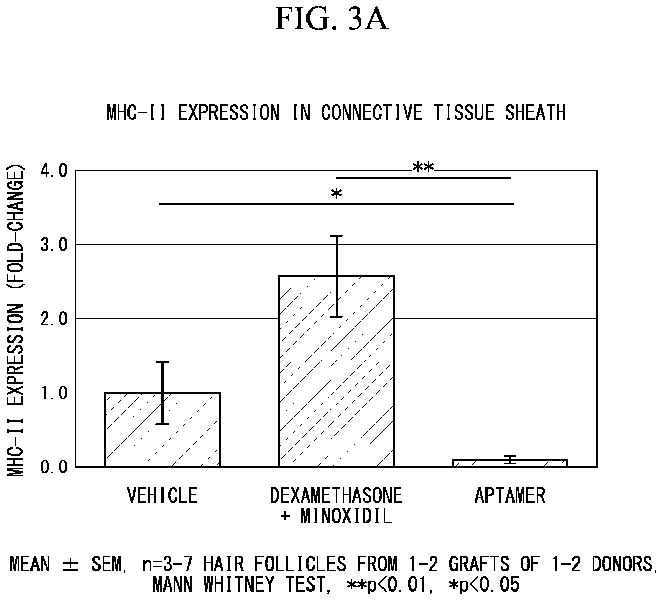

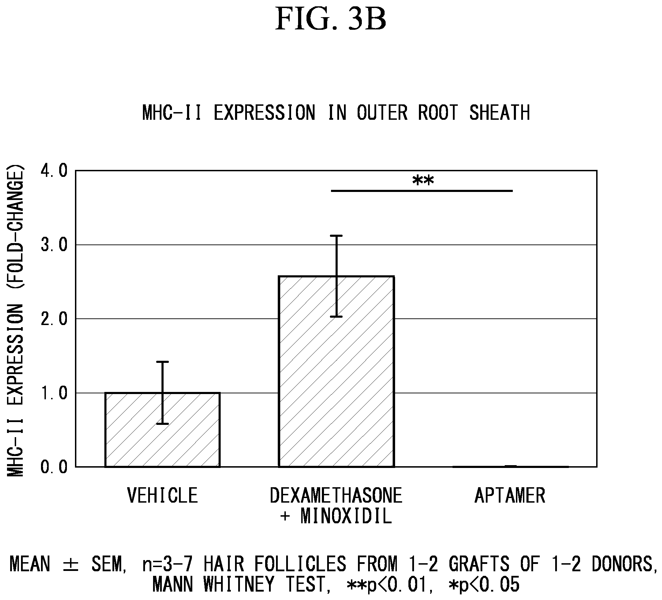

Further, the expression of MHC was examined by the pathological analysis of the hair follicle tissue after the administration in Example 4. Fig. 2A shows the results of expression of MHC class I in the dermal sheath cups. Fig. 2B shows the results of expression of MHC class I in the outer root sheaths. Fig. 3A shows the results of expression of MHC class II in the connective tissue sheaths. Fig. 3B shows the results of expression of MHC class II in the outer root sheaths. The vertical axis shows the expression levels of MHC class I (Fig. 2A and Fig. 2B) or class II (Fig. 3A and Fig. 3B) as relative values to the expression level in the respective Vehicle group ("Vehicle" in Fig. 2A, Fig. 2B, Fig. 3A and Fig. 3B) as 1.

Inhibition of expression of both MHC class I and II was observed only in the Aptamer-treated group ("Aptamer" in Fig. 2A, Fig. 2B, Fig. 3A and Fig. 3B). This indicates that the mechanism which suppressed inflammation and promoted hair regeneration was different between the Positive control group ("Dexamethasone + Minoxidil" in Fig. 2A, Fig. 2B, Fig. 3A and Fig. 3B) and the Aptamer-treated group. It is assumed that the Positive control group showed direct suppression of inflammation by the activation of glucocorticoid receptors, while the Aptamer-treated group suppressed inflammation by inhibiting the expression of MHC class I and class II, which cause autoimmunity. In other words, in the Aptamer-treated group, hair follicle tissues recovered from the immune privilege collapse, suggesting that a more fundamental therapeutic efficacy was obtained. This result suggests that the DNA aptamer of the present embodiment may suppress inflammatory reactions not only in autoimmune skin diseases, but also in autoimmune diseases that occur in other tissues and in diseases such as Hunner-type interstitial cystitis resulting mainly from the overproduction of IFN-γ, through similar mechanisms.

It was confirmed that the use of the therapeutic agent containing the DNA aptamer of the present embodiment could suppress hair loss and also promote hair regeneration. Further, as a result of the pathological analysis, it was confirmed that the administration of the DNA aptamer of the present embodiment could almost completely inhibit the expression of MHC class I and II.

### Example 5: Generation of mouse IFN-γ aptamer (surrogate aptamer)

### Step 1

The aptamer was obtained by SELEX method targeting mouse IFN-γ. The obtained mouse IFN-γ aptamer is a DNA aptamer having a sequence listed in SEQ ID NO: 4, having residues with 62 bases, containing two Ds, and having a mini-hairpin sequence on the 3' end thereof (Aptamer 4 in Table 1). The affinity to mouse IFN-γ was measured by SPR analysis, and confirmed the KD value was 2.47 nM.

### Step 2

It was verified that the obtained aptamer inhibited the activity of mouse IFN-γ. First, 2 ng/mL mouse IFN-γ and each of the Aptamer 4 solutions at various molar concentrations were simultaneously added to L929 mouse fibroblasts, followed by incubation at 37°C for 15 minutes. Then, the inhibition of STAT1 phosphorylation by the Aptamer 4 was confirmed by flow cytometry analysis using an anti-phosphorylated STAT1 antibody.

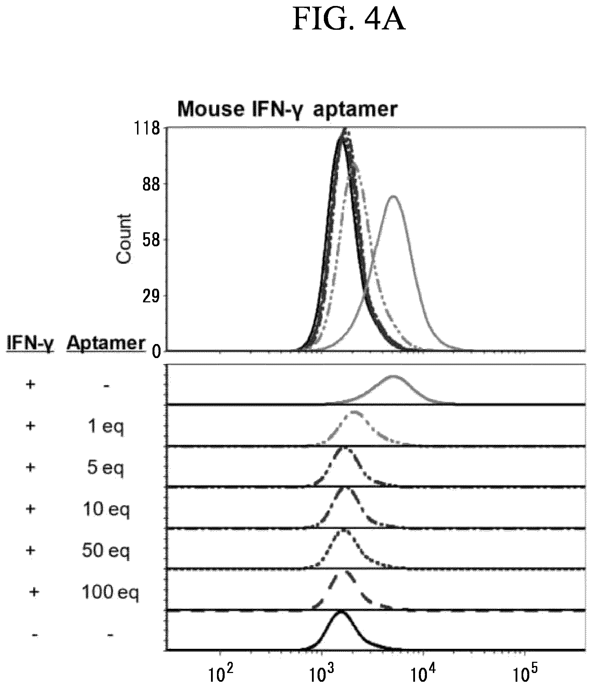

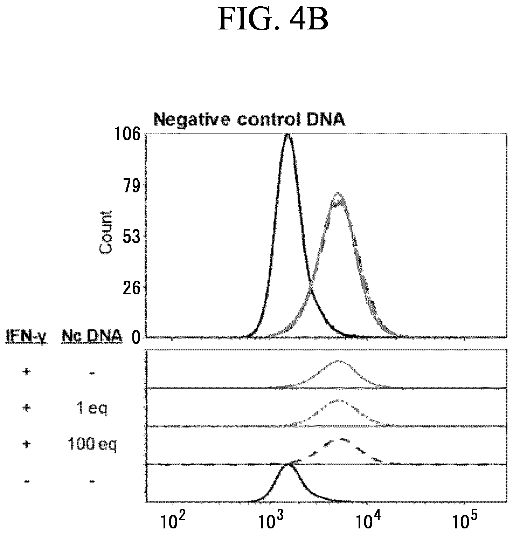

Figs. 4A and 4B show the results. Fig. 4A shows the results of addition of the Aptamer 4 to L929 mouse fibroblasts. Fig. 4B shows the results of addition of the negative control DNA to L929 mouse fibroblasts. In Fig. 4A, the term "Aptamer" means the surrogate aptamer of the present embodiment. In Fig. 4B, the term "Nc DNA" means the negative control DNA. In Figs. 4A and 4B, the terms "1 eq", "5 eq", "10 eq", "50 eq", and "100 eq" indicate the ratios of the molar concentration of the aptamer to the molar concentration of IFN-γ (for example, 100 eq indicates that aptamer:IFN-γ = 100:1). The addition of Aptamer 4 five times the molar concentration of IFN-γ inhibited the phosphorylation of STAT1 almost completely (Fig. 4A). Meanwhile, even though the negative control DNA was added to IFN-γ at 100 times amount of mouse IFN-γ in the system to which the negative control DNA was added, the phosphorylation of STAT1 was not inhibited (Fig. 4B). This result confirmed that the obtained Aptamer 4 inhibited the activity of mouse IFN-γ, and had sufficient activity as a surrogate aptamer.

### Example 6: Examination of the effect of surrogate aptamer in mouse autoimmune interstitial cystitis model

### Step 1 Generation of autoimmune interstitial cystitis model (URO-OVA model) mice

Autoimmune interstitial cystitis model (URO-OVA model) mice were generated in accordance with the method described in Reference Literature 1. Normal mice were subcutaneously injected with 100 µg of an OVA antigen, and the splenocytes were collected from the immunized mice after two weeks. Then, 5 × 10^7 cells of the splenocytes were transplanted into each transgenic mouse expressing the OVA antigen at the bladder epithelium (URO-OVA mouse) by intravenous injection. In the URO-OVA mice which the splenocytes were transplanted, the immunoreaction specifically at the bladder epithelium was induced, resulting in inflammation.

### Step 2

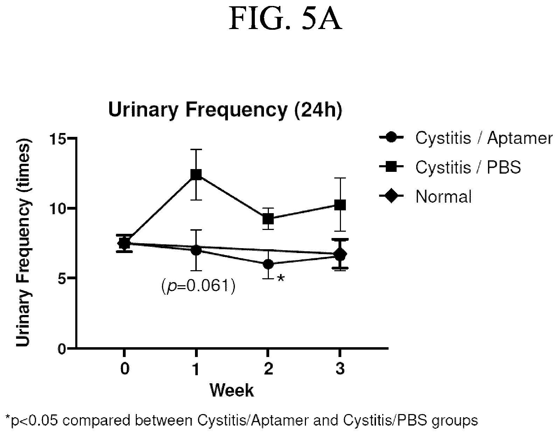

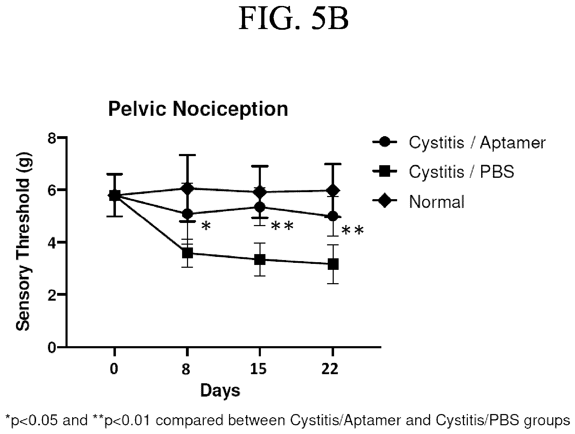

The surrogate aptamer was intravesically administered to each autoimmune interstitial cystitis model (URO-OVA model) mouse once every two days over three weeks (10 nmol per one time). The PBS-treated group (interstitial cystitis model mice) and the Normal group (normal mice) were used as the control groups. Neither surrogate aptamer nor PBS was administered to the Normal group (normal mice). Fig. 5A shows the results of the frequency of urination for 24 hours once a week. While the PBS-treated group (interstitial cystitis model mice) (■) significantly increased in urinary frequency compared to the Normal group (◆), the increase in urinary frequency was completely suppressed in the aptamer-treated group (•), and the urinary frequency of the aptamer-treated mice was equivalent to those of the normal mice. Fig. 5B shows the results of the evaluation of the sensitivity to pelvic nociception by an Electronic Von Frey once every seven days. While the PBS-treated group (interstitial cystitis model mice) (■) significantly increased in sensitivity to pelvic nociception (decreased in sensory threshold) compared to the Normal group (◆), the increase in sensitivity was significantly suppressed in the aptamer-treated group (•). The above results showed that the increase in the urinary frequency of the mice (Fig. 5A) and the increase in sensitivity to pelvic nociception (Fig. 5B) were significantly suppressed in the aptamer-treated group compared to those in the PBS-treated group, the aptamer exhibited a high efficacy of suppressing the onset of cystitis.

### Step 3

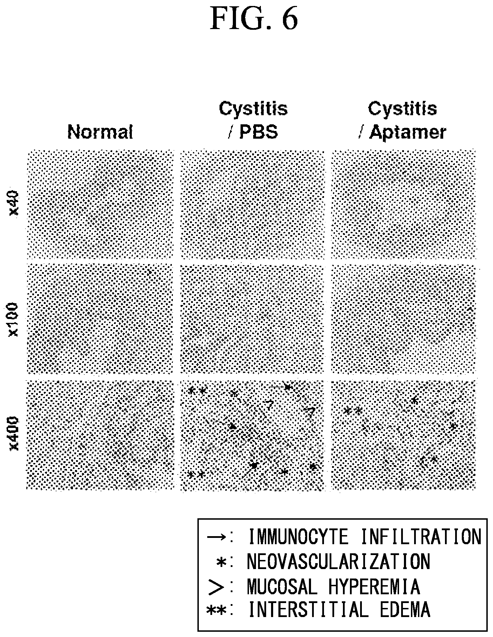

The mice were sacrificed, followed by histopathological evaluation of the bladders after the observation period for three weeks from the initiation of administration of the aptamer or PBS. Fig. 6 and Table 2 show the results. As a result of HE staining, immune cell infiltration (→), angiogenesis (*), mucosal hyperemia (>), and interstitial edemas (**) were observed in the PBS-treated group, and almost none of these changes were meanwhile observed in the aptamer-treated group. Table 2 shows the bladder inflammation scores after the observation period. Grade 0 indicates no findings of inflammation. As the grade increases from Grade 1 to Grade 3, observed findings of inflammation such as immune cell infiltration, angiogenesis, mucosal hyperemia, and interstitial edemas increases. Since the aptamer-treated group ("Cystitis/Aptamer" in Table 2) is significantly low in inflammation score compared to the PBS-treated group ("Cystitis/PBS" in Table 2), and is not different therein from the Normal group ("Normal" in Table 2), it was shown that the aptamer administration suppressed the bladder inflammation.

**[Table 2]**

| Bladder Inflammatory Score | | | | |
|---|---|---|---|---|
| | Grade 0 | Grade 1 | Grade 2 | Grade3 |
| Normal (n = 5) | 3 | 2 | | |
| Cystitis / PBS (n = 5) | | 2 | I | 2 |
| Cystitis / Aptamer (n = 7) | 2 | 5 | | |

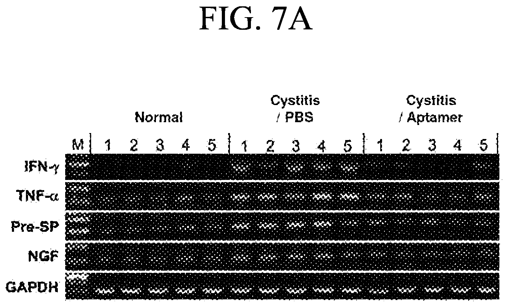

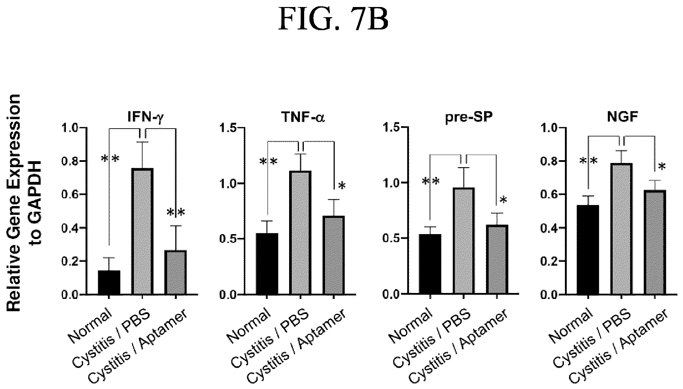

Figs. 7A and 7B show the results of mRNA expression analysis in bladder tissue. Fig. 7A shows the results of 1% agarose gel electrophoresis of RT-PCR products. Fig. 7B shows the graph quantifying the expression level of mRNA shown in Fig. 7A. In the surrogate aptamer-treated group ("Cystitis/Aptamer" in Figs. 7A and 7B), the aptamer reduced the expression levels of IFN-γ and TNF-α, which were inflammatory cytokines induced by IFN-γ stimulation, and the expression levels of pre-SP and NGF, which functioned as pain-causing substances, in bladder tissue to approximately the normal levels, which were equivalent to those of the Normal group. This strongly suggested that the effects of the administration of the surrogate aptamer on the urinary frequency, the sensitivity to pelvic nociception, and bladder inflammation were to be due to inhibition of IFN-γ activity in the tissue.

It is thought that the results in Examples 4 and 6 above may have resulted from the strong inhibition of IFN-γ activity by the DNA aptamer of the present embodiment. Therefore, the DNA aptamer of the present embodiment can provide an unprecedented and effective therapeutic agent and treatment method for autoimmune diseases, including alopecia areata, and diseases such as Hunner-type interstitial cystitis resulting mainly from the overproduction of IFN-γ.

From the results of the above Examples, it is also thought that the DNA aptamer of the present embodiment binds to IFN-γ with high specificity. When compared to Janus kinase inhibitors which inhibit multiple cytokine signals due to its pharmacological properties (Yvan Jamilloux, et al., Autoimmunity Reviews, 2019, 18, 102390), the DNA aptamer of the present embodiment which selectively inhibits IFN-γ activity can reduce the possibility of occurrence of side effects.

DNA aptamers are generally unlikely to produce anti-DNA aptamer antibodies. This allows the DNA aptamer of the present embodiment to be administered over a long term in the treatment of chronic inflammatory diseases.

### Example 7: Confirmation of binding of DNA aptamer of the present embodiment to dog IFN-γ and cat IFN-γ

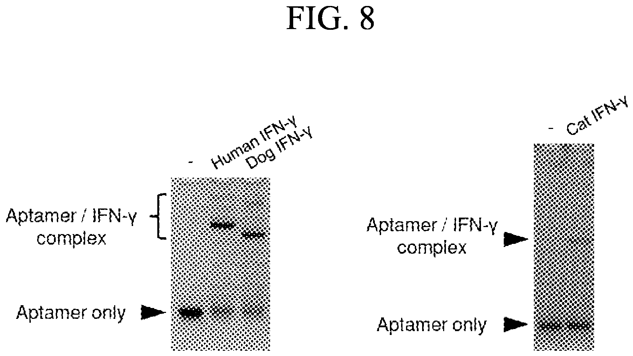

Electrophoretic mobility shift assay (EMSA) confirmed the binding of the DNA aptamer of the present embodiment to dog IFN-γ and cat IFN-γ. First, 100 nM Aptamer 2 was mixed with 400 nM dog IFN-γ or cat IFN-γ, and the mixture was left at room temperature for 15 to 30 minutes. The obtained samples were subjected to 8 to 10% polyacrylamide gel electrophoresis, followed by detection with SYBR Gold. Fig. 8 shows the results. Since a shift band indicating the complex of Aptamer 2 and dog IFN-γ (left in Fig. 8) and a shift band indicating the complex of Aptamer 2 and cat IFN-γ (right in Fig. 8) were detected, it was confirmed that Aptamer 2 binds to both dog IFN-γ and cat IFN-γ. A test for confirming the binding of Aptamer 2 to human IFN-γ was simultaneously performed by the test method described above as a positive control for the EMSA. As a result, a shift band indicating the complex was detected, so that the binding of Aptamer 2 to human IFN-γ was confirmed.

It is presumed from the results in the above-mentioned Example 7 that the DNA aptamer of the present embodiment inhibits the activities of dog IFN-γ and cat IFN-γ. Accordingly, the use of the DNA aptamer enables providing a therapeutic agent and a therapy effective against idiopathic cystitis, which is one of the lower urinary tract diseases resulting from the bladders of dogs and cats, and diseases and autoimmune diseases related to IFN-γ.

The DNA aptamer of the present embodiment can be produced by chemical synthesis. Therefore, it can be provided as a safe agent with stable quality and low risk of biological contamination.

The DNA aptamer of the present embodiment can be produced at a lower cost than biologics. Moreover, while biologics require low-temperature conditions for the storage and transportation of biologics, DNA aptamers are stable at room temperature. Therefore, a cold chain is not necessarily required in the transportation and storage of the drug formulation containing the DNA aptamer of the present embodiment.

By formulating the DNA aptamer of the present embodiment into a transdermal administration formulation, a therapeutic agent which is not invasive in administration, is less concern for adverse effects and is easy to use can be provided.

The therapeutic agent containing the DNA aptamer of the present embodiment can be indicated for systemic autoimmune diseases and diseases such as Hunner-type interstitial cystitis resulting mainly from the overproduction of IFN-γ by systemic administration of it as an injectable formulation. Moreover, when made into an injectable formulation, it can be a drug formulation which is easy to use for patients, such as a pre-filled syringe which can be stored at room temperature.

Since the DNA aptamer of the present embodiment can selectively inhibit IFN-γ, the DNA aptamer is available as a research reagent for experiment on systems in which IFN-γ may be involved. For example, regardless of *in vitro* or *in vivo,* the possibility whether IFN-γ is involved in a focused physiological phenomenon can be evaluated and examined by the test using the DNA aptamer of the present embodiment, to consider the cause of the physiological phenomenon. The DNA aptamer of the present embodiment is added as a reagent to cell culture medium or administered to animals to be available for a wide variety of tests including reaction systems in which IFN-γ is inhibited.

## Claims

1. A Hunner-type interstitial cystitis therapeutic agent comprising as an active ingredient a DNA oligonucleotide having a nucleotide sequence set forth in any of SEQ ID NO:1 to 3 and selectively binding to interferon-y (IFN-γ).

2. The Hunner-type interstitial cystitis therapeutic agent according to claim 1, wherein a base, X, in a sequence of the DNA oligonucleotide having the nucleotide sequence set forth in SEQ ID NO: 3 is an artificially produced base, and the artificially produced base is chemically modified with a low-molecular-weight compound.

3. The Hunner-type interstitial cystitis therapeutic agent according to claim 2, wherein the low-molecular-weight compound is an anti-inflammatory compound selected from glucocorticoid, tacrolimus, sirolimus, cyclosporine, methotrexate and leflunomide.

4. The Hunner-type interstitial cystitis therapeutic agent according to claim 1, wherein a base X in a sequence of the DNA oligonucleotide having a nucleotide sequence set forth in SEQ ID NO:3 is an artificially produced base, and the artificially produced base is chemically modified with a medium-molecular-weight compound, a high-molecular-weight compound, a biopolymer, or a biocompatible polymer.

5. The Hunner-type interstitial cystitis therapeutic agent according to claim 4, wherein the high-molecular-weight compound is a polyethylene glycol (PEG) with a molecular weight of 20000 or more or any biocompatible large molecule with a molecular weight of 20000 or more.

6. A therapeutic agent comprising as an active ingredient a DNA oligonucleotide having a nucleotide sequence set forth in any of SEQ ID NO:1 to 3 and selectively binding to dog IFN-γ and cat IFN-y, wherein the therapeutic agent is used for a disease selected from diseases related to dog IFN-γ and cat IFN-γ, lower urinary tract diseases due to bladders of dogs and cats, and autoimmune diseases of dog and cat.

7. The therapeutic agent according to claim 6, wherein a base, X, in the sequence of the DNA oligonucleotide having the nucleotide sequence set forth in SEQ ID NO: 3 is an artificially produced base, and the artificially produced base is chemically modified with a low-molecular-weight compound.

8. The therapeutic agent according to claim 6, wherein a base, X, in the sequence of the DNA oligonucleotide having the nucleotide sequence set forth in SEQ ID NO: 3 is an artificially produced base, and the artificially produced base is chemically modified with a medium-molecular-weight compound, a high-molecular-weight compound, a biopolymer, or a biocompatible polymer.

9. A reagent for test and research, comprising as an active ingredient a DNA oligonucleotide having a nucleotide sequence set forth in any of SEQ ID NO: 1 to 3 and selectively binding to IFN-γ.

10. The reagent for test and research according to claim 9, wherein a base, X, in the sequence of the DNA oligonucleotide having the nucleotide sequence set forth in SEQ ID NO: 3 is an artificially produced base, and the artificially produced base is chemically modified with a low-molecular-weight compound.

11. The reagent for test and research according to claim 9, wherein a base, X, in the sequence of the DNA oligonucleotide having the nucleotide sequence set forth in SEQ ID NO: 3 is an artificially produced base, and the artificially produced base is chemically modified with a medium-molecular-weight compound, a high-molecular-weight compound, a biopolymer, or a biocompatible polymer.
